(19) **Europäisches Patentamt / European Patent Office / Office européen des brevets**

(11) **EP 2 565 031 A1**

(12) # EUROPEAN PATENT APPLICATION
published in accordance with Art. 153(4) EPC

(43) Date of publication:
06.03.2013 Bulletin 2013/10

(51) Int Cl.:
*B32B 27/12* [(2006.01)]   *A61F 13/15* [(2006.01)]
*A61F 13/49* [(2006.01)]   *A61F 13/53* [(2006.01)]
*B32B 5/26* [(2006.01)]

(21) Application number: 11774870.7

(22) Date of filing: 19.04.2011

(86) International application number:
PCT/JP2011/059626

(87) International publication number:
WO 2011/136087 (03.11.2011 Gazette 2011/44)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 30.04.2010 JP 2010104892

(71) Applicant: Sumitomo Seika Chemicals Co. Ltd.
Hyogo 675-0145 (JP)

(72) Inventors:
• FUKUDOME, Shinya
Hyogo 672-8076 (JP)

• TAKATORI, Junichi
Hyogo 672-8076 (JP)
• MARUO, Junichi
Hyogo 672-8076 (JP)
• HINAYAMA, Tetsuhiro
Hyogo 672-8076 (JP)

(74) Representative: Jackson, Martin Peter et al
J A Kemp
14 South Square
Gray's Inn
GB-London WC1R 5JJ (GB)

(54) **WATER-ABSORBING SHEET STRUCTURE**

(57) A water-absorbent sheet structure comprising a structure in which an absorbent layer containing a water-absorbent resin and an adhesive is sandwiched with nonwoven fabrics from an upper side and a lower side of the absorbent layer, wherein the water-absorbent sheet structure has a structure in which the absorbent layer is separated in divided parts of a primary absorbent layer and a secondary absorbent layer with a fibrous substrate having a void ratio of from 91 to 99%, and wherein the water-absorbent resin is contained in an amount of from 100 to 1,000 g/m$^2$, and wherein the adhesive is contained in a proportion of 0.05 to 2.0 times the amount of the water-absorbent resin contained (mass basis). The water-absorbent sheet structure of the present invention exhibits some effects that a water-absorbent sheet structure has excellent shape retaining ability even when the structure is thin, so that the water-absorbent sheet structure does not undergo deformation of the form before liquid absorption or after the absorption, and is capable of sufficiently exhibiting absorbent properties such as liquid permeability, small amount of re-wet, and small slope liquid leakage.

**FIG. 1**

EP 2 565 031 A1

**Description**

**TECHNICAL FIELD**

The present invention relates to a water-absorbent sheet structure which can be used in the fields of hygienic materials and the like. More specifically, the present invention relates to a water-absorbent sheet structure which is thin and can be suitably used in absorbent articles, such as disposable diapers. In addition, the present invention relates to an absorbent article such as disposable diapers using the water-absorbent sheet structure.

**BACKGROUND ART**

Absorbent articles represented by disposable diapers or the like have a structure in which an absorbent material for absorbing a liquid such as a body liquid is sandwiched with a flexible liquid-permeable surface sheet (top sheet) positioned on a side contacting a body and a liquid-impermeable backside sheet (back sheet) positioned on a side opposite to that contacting the body.

Conventionally, there have been increasing demands for thinning and light-weighing of absorbent articles, from the viewpoint of designing property, convenience upon carrying, and efficiency upon distribution. Further, in the recent years, there have been growing needs for so-called eco-friendly intentions, in which resources are effectively utilized so that use of natural materials that require a long time to grow such as trees is avoided as much as possible, from the viewpoint of environmental protection. Conventionally, a method for thinning that is generally carried out in absorbent articles is, for example, a method of reducing hydrophilic fibers such as crushed pulp of a wood material, which has a role of fixing a water-absorbent resin in an absorbent material, while increasing a water-absorbent resin.

An absorbent material in which a water-absorbent resin having a smaller volume and higher water-absorbent capacity is used in a large amount with a lowered proportion of a hydrophilic fiber being bulky and having lower water-absorbent properties is intended to achieve thinning by reducing bulky materials while obtaining absorption capacity matching the design of an absorbent article, so that it is considered as a reasonable improved method. However, when distribution or diffusion of a liquid upon actually using in an absorbent article such as disposable diapers is considered, there is a disadvantage that if a large amount of the water-absorbent resin is formed into a soft gel-like state by absorption of the liquid, a so-called "gel-blocking phenomenon" takes place, whereby liquid diffusibility is markedly lowered and a liquid permeation rate of the absorbent material is slowed down. This "gel-blocking phenomenon" is a phenomenon in which especially when an absorbent material in which water-absorbent resins are highly densified absorbs a liquid, water-absorbent resins existing near a surface layer absorb the liquid to form soft gels that are even more densified near the surface layer, so that a liquid permeation into an internal of an absorbent material is blocked, thereby making the internal of the water-absorbent resin incapable of efficiently absorbing the liquid.

In view of the above, conventionally, as a means of inhibiting gel-blocking phenomenon which takes place by reducing hydrophilic fibers while using a water-absorbent resin in a large amount, for example, proposals such as a method using an absorbent polymer having such properties as specified Saline Flow Conductivity and Performance under Pressure (see Patent Publication 1), and a method using a water-absorbent resin prepared by heat-treating a specified water-absorbent resin precursor with a specified surface crosslinking agent (see Patent Publication 2) have been made.

However, in these methods, the liquid absorbent properties as absorbent materials in which water-absorbent resins are used in large amounts are not satisfactory. In addition, there arise some problems that the water-absorbent resin is subjected to be mobile before use or during use because hydrophilic fibers that play a role of fixing the water-absorbent resin are reduced. The absorbent material in which the localization of the absorbent resin takes place is more likely to cause gel-blocking phenomenon.

Further, an absorbent material of which hydrophilic fibers that contribute to retention of the form are reduced has a lowered shape retaining ability as an absorbent material, so that deformation in shapes such as twist-bending or tear before or after the absorption of a liquid is likely to take place. An absorbent material with deformation in shapes has markedly lowered liquid diffusibility, so that abilities inherently owned by the absorbent material cannot be exhibited. In order to try to avoid such phenomena, a ratio of hydrophilic fibers and a water-absorbent resin would be limited, thereby posing limitations in the thinning of an absorbent article.

In view of the above, in recent years, as a next generation style absorbent material which is capable of increasing a content of a water-absorbent resin while using hydrophilic fibers in an absorbent material as little as possible, studies have been widely made on an absorbent laminate that substantially does not contain hydrophilic fibers in an absorbent layer, a water-absorbent sheet or the like. The studies include, for example, a method of keeping a water-absorbent resin in reticulation of a bulky nonwoven fabric (see Patent Publication 3), a method of sealing a water-absorbent polymer between two sheets of meltblown nonwoven fabrics (see Patent Publication 4), a method of interposing water-absorbent polymer particles between a hydrophobic nonwoven fabric and a hydrophilic sheet (see Patent Publication 5), and the like.

However, in a case where hydrophilic fibers are hardly used, the gel-blocking phenomenon as mentioned above is likely to take place. Even in a case where gel-blocking phenomenon does not take place, a thing that would serve the role of conventional hydrophilic fibers by which a body fluid such as urine is temporarily subjected to water retention and diffusion of the liquid to an overall absorbent material is lacking, so that a liquid leakage is likely to occur in the absorbent laminate, without being able to sufficiently capture the liquid.

Further, when an adhesive is used for retaining the shape of an absorbent laminate, the surface of a water-absorbent resin is coated with an adhesive, so that liquid absorbent properties are likely to be lowered. Alternatively, an upper side and a lower side of nonwoven fabrics are firmly adhered with an adhesive to confine an water-absorbent resin in a pouched form or the like, so that the water-absorbent properties inherently owned by the water-absorbent resin are less likely to be exhibited.

When adhesive strength of an absorbent laminate is weakened in order to improve liquid absorbent properties of the above-mentioned absorbent laminate, not only a large amount of the absorbent resin is detached upon working on the laminate, thereby making unfavorable economically, but also the laminate is exfoliated due to deficiency in adhesive strength, so that there are some possibilities of loss of commercial values. In other words, if the adhesion is strengthened, the gel-blocking phenomenon or liquid leakage occurs, and if the adhesion is weakened, it would lead to the detachment of a water-absorbent resin and the breaking of the laminate, so that an absorbent laminate or a water-absorbent sheet for which the above-mentioned problems are solved is not yet obtained at present.

Studies on improvement of the balance between adhesion and the liquid absorbent properties in the water-absorbent sheets as described above are also made. The studies include, for example, a method of using an absorbent laminate comprising two sheets of nonwoven fabrics adhered with reticular layers provided between the nonwoven fabrics, comprising upper and lower two layers of hot melt adhesives (see Patent Publication 6), a method of applying a specified reactive hot melt to a substrate made of a nonwoven fabric or a film, thereby fixing a water-absorbent resin (see Patent Publication 7), a method of coating a fine cellulose and a water-absorbent resin with a network-like hot melt to hold them (see Patent Publication 8), and the like. However, even if properties of a nonwoven fabric, a water-absorbent resin, and an adhesive, or conditions of use thereof are defined, it is difficult to obtain a water-absorbent sheet having high liquid absorbent properties and shape retaining ability. In addition, if a specified adhesive or method of adhesion is used, it is not desirable from the viewpoint of economical advantages and productivity, even if the liquid absorbent properties were improved.

There is also a method of immobilizing a water-absorbent resin to a substrate without using an adhesive. The method includes, for example, a method of adhering water-absorbent polymer particles in the process of polymerization to a synthetic fibrous substrate to carry out polymerization on the fibrous substrate (see Patent Publication 9), a method of polymerizing a monomer aqueous composition containing acrylic acid and an acrylic acid salt as main components on a nonwoven fabric substrate by means of electron beam irradiation (see Patent Publication 10), and the like.

In these methods, while the synthetic fibrous substrate is penetrated into the polymer particles to be firmly adhered, there are some disadvantages that it is difficult to complete the polymerization reaction in the substrate, so that unreacted monomers and the like remain in the substrate in large amounts.

## PRIOR ART PUBLICATIONS

## PATENT PUBLICATIONS

Patent Publication 1: Japanese Unexamined Patent Publication No. Hei-9-510889
Patent Publication 2: Japanese Patent Laid-Open No. Hei-8-057311
Patent Publication 3: Japanese Unexamined Patent Publication No. Hei-9-253129
Patent Publication 4: Japanese Patent Laid-Open No. Hei-7-051315
Patent Publication 5: Japanese Patent Laid-Open No. 2002-325799
Patent Publication 6: Japanese Patent Laid-Open No. 2000-238161
Patent Publication 7: Japanese Unexamined Patent Publication No. 2001-158074
Patent Publication 8: Japanese Unexamined Patent Publication No. 2001-096654
Patent Publication 9: Japanese Patent Laid-Open No. 2003-011118
Patent Publication 10: Japanese Patent Laid-Open No. Hei-2-048944

## SUMMARY OF THE INVENTION

### PROBLEMS TO BE SOLVED BY THE INVENTION

In view of the above, an object of the present invention is to provide a water-absorbent sheet structure which is capable of avoiding the gel-blocking phenomenon even when the water-absorbent sheet structure contains a very small amount of pulps, so that the water-absorbent sheet structure has excellent fundamental properties (fast liquid permeation rate, small amount of liquid re-wet, small liquid leakage, and shape retaining ability), and is capable of accomplishing thinning.

### MEANS TO SOLVE THE PROBLEMS

Specifically, the gist of the present invention relates to:

[1] a water-absorbent sheet structure comprising a structure in which an absorbent layer containing a water-absorbent resin and an adhesive is sandwiched with a nonwoven fabric from an upper side and a lower side of the absorbent layer, wherein the water-absorbent sheet structure has a structure in which the absorbent layer is separated in divided parts of a primary absorbent layer and a secondary absorbent layer with a fibrous substrate having a void ratio of from 91 to 99%, and wherein the water-absorbent resin is contained in an amount of from 100 to 1,000 g/m$^2$, and wherein the adhesive is contained in a ratio of 0.05 to 2.0 times the amount of the water-absorbent resin contained (mass basis); and
[2] an absorbent article comprising the water-absorbent sheet structure as defined in the above [1], sandwiched between a liquid-permeable sheet and a liquid-impermeable sheet.

### EFFECTS OF THE INVENTION

The water-absorbent sheet structure of the present invention exhibits some effects that a water-absorbent sheet structure has excellent shape retaining ability even when the structure is thin, so that the water-absorbent sheet structure does not undergo deformation of the form before liquid absorption or after the absorption, and is capable of sufficiently exhibiting absorbent properties such as liquid permeability, small amount of liquid re-wet, and small slope liquid leakage. Therefore, the water-absorbent sheet structure according to the present invention is used for an absorbent material such as disposable diapers, whereby hygienic materials which are thin and have excellent design property, and at the same time not having disadvantages such as gel-blocking phenomena and liquid leakage can be provided. Also, the water-absorbent sheet structure according to the present invention can be used in agricultural fields and fields of construction materials other than the field of hygienic materials.

### BRIEF DESCRIPTION OF THE DRAWINGS

[Figure 1]   A cross-sectional schematic view of one embodiment of a water-absorbent sheet structure according to the present invention.
[Figure 2]   A cross-sectional schematic view of another embodiment of a water-absorbent sheet structure according to the present invention.
[Figure 3]   A cross-sectional schematic view of another embodiment of a water-absorbent sheet structure according to the present invention.
[Figure 4]   A schematic view showing an outline of the constitution of an instrument used for measuring strength for a water-absorbent sheet structure.
[Figure 5]   A schematic view showing an outline of the constitution of an apparatus used for carrying out a slope leakage test for a water-absorbent sheet structure.

### MODES FOR CARRYING OUT THE INVENTION

The water-absorbent sheet structure according to the present invention is a water-absorbent sheet structure comprising a structure in which an absorbent layer containing a water-absorbent resin and an adhesive is sandwiched with nonwoven fabrics. Since the water-absorbent sheet structure has a structure in which the absorbent layer is separated in divided parts of a primary absorbent layer and a secondary absorbent layer with a fibrous substrate having a void ratio in a specified range, and the absorbent layer in which the water-absorbent resin and the adhesive are used in given amounts is formed between the nonwoven fabrics, a thin water-absorbent sheet structure also having excellent liquid absorbent properties such as liquid permeability, small amount of liquid re-wet, and small slope liquid leakage can be realized.

Further, in the water-absorbent sheet structure according to the present invention, a water-absorbent resin is firmly adhered to nonwoven fabrics with an adhesive, localization or scattering of the water-absorbent resin can be prevented, even when the water-absorbent resin substantially does not contain a hydrophilic fiber such as pulp fiber, and the shape retaining ability can also be favorably maintained. Also, since the water-absorbent sheet structure has a peeling strength in a specified range, the water-absorbent resin is not in a state where the entire surface is coated with an adhesive but a state that a part thereof is firmly adhered, so that it is considered that the water-absorbent resin can be sufficiently swollen, while the water-absorbent properties of the water-absorbent resin are hardly inhibited.

The water-absorbent sheet structure according to the present invention may be in an embodiment where a hydrophilic fiber such as pulp fiber is admixed between the nonwoven fabrics together with the water-absorbent resin in an amount that would not impair the effects of the present invention. However, it is preferable that the structure is in an embodiment where a hydrophilic fiber is substantially not contained, from the viewpoint of thinning.

As the kinds of the above-mentioned water-absorbent resins, commercially available water-absorbent resins can be used. For example, the water-absorbent resin includes hydrolysates of starch-acrylonitrile graft copolymers, neutralized products of starch-acrylic acid graft polymers, saponified products of vinyl acetate-acrylic acid ester copolymers, partially neutralized products of polyacrylic acid, and the like. Among these water-absorbent resins, the partially neutralized products of polyacrylic acids are preferred, from the viewpoint of production amount, production costs, water-absorbent properties, and the like. Methods for synthesizing partially neutralized products of polyacrylic acid include reversed phase suspension polymerization method, aqueous solution polymerization method, and the like. Among these polymerization methods, the water-absorbent resins obtained according to reversed phase suspension polymerization method are preferably used, from the viewpoint of excellent flowability of the resulting particles, smaller amounts of fine powder, high water-absorbent properties, such as liquid absorption capacity (expressed by indices such as water-retention capacity, water-absorption capacity, water-absorption capacity under load), and water-absorption rate.

The partially neutralized product of a polyacrylic acid has a degree of neutralization of preferably 50% by mol or more, and even more preferably from 70 to 90% by mol, from the viewpoint of increasing an osmotic pressure of the water-absorbent resin, thereby increasing water-absorbent properties.

The water-absorbent resin is contained in the water-absorbent sheet structure, in a total amount of a primary absorbent layer and a secondary absorbent layer, of from 100 to 1,000 g per one square-meter of the water-absorbent sheet structure, i.e. 100 to 1,000 g/m$^2$, preferably from 150 to 800 g/m$^2$, more preferably from 200 to 700 g/m$^2$, and even more preferably from 220 to 600 g/m$^2$, from the viewpoint of obtaining sufficient liquid absorbent properties even when a water-absorbent sheet structure according to the present invention is used for an absorbent article. The water-absorbent resin is contained in an amount of 100 g/m$^2$ or more, from the viewpoint of exhibiting sufficient liquid absorbent properties as a water-absorbent sheet structure, thereby suppressing especially re-wetting, and the water-absorbent resin is contained in a total amount of 1,000 g/m$^2$ or less, from the viewpoint of suppressing the gel-blocking phenomenon from being caused, exhibiting liquid diffusibility as a water-absorbent sheet structure, and further improving a liquid permeation rate.

The water-absorbent resin ratio (mass ratio) of the primary absorbent layer/secondary absorbent layer mentioned above is preferably within the range of from primary absorbent layer/secondary absorbent layer = 98/2 to 50/50, more preferably with the range of from primary absorbent layer/secondary absorbent layer = 98/2 to 60/40, even more preferably within the range of from primary absorbent layer/secondary absorbent layer = 95/5 to 70/30, and still even more preferably within the range of from primary absorbent layer/secondary absorbent layer = 95/5 to 80/20. The ratio of the primary absorbent layer/secondary absorbent layer is preferably 98 or less/2 or more, from the viewpoint of sufficiently exhibiting water-absorbent properties of a secondary absorbent layer, thereby preventing liquid leakage, and the ratio of the primary absorbent layer/secondary absorbent layer is preferably in a ratio of 50 or more/50 or less, from the viewpoint of increasing dry feel of the primary absorbent layer and lowering the amount of re-wet after liquid absorption.

The liquid absorbent properties of the water-absorbent sheet structure according to the present invention are influenced by the water-absorbent properties of the water-absorbent resin used. Therefore, it is preferable that the water-absorbent resin of the primary absorbent layer and the secondary absorbent layer to be used in the present invention is those selected with favorable ranges in water-absorbent properties such as liquid absorption capacity (expressed by indices such as water-retention capacity), and water-absorption rate, and mass-average particle size of the water-absorbent resin, by taking the constitution of each component of the water-absorbent sheet structure or the like into consideration. Therefore, the kinds of the water-absorbent resin of the primary absorbent layer and the kinds of the water-absorbent resin of the secondary absorbent layer may be identical to or different from each other.

In the present specification, the water-retention capacity of the water-absorbent resin is evaluated as a water-retention capacity of saline solution. The water-absorbent resin has a water-retention capacity of saline solution of preferably 25 g/g or more, more preferably from 25 to 60 g/g, and even more preferably from 30 to 50 g/g, from the viewpoint of absorbing a liquid in a larger amount, and preventing the gel-blocking phenomenon while keeping the gel strong during absorption. The water-retention capacity of saline solution of the water-absorbent resin is a value obtainable by a measurement method described in Examples set forth below.

In the present specification, the water-absorption rate of the water-absorbent resin is evaluated as a water-absorption rate of saline solution. The water-absorbent resin has a water-absorption rate of saline solution of preferably from 2 to 70 seconds, more preferably from 3 to 60 seconds, and even more preferably from 3 to 55 seconds, from the viewpoint of speeding up the liquid permeation rate of the water-absorbent sheet structure according to the present invention, thereby preventing a liquid leakage upon use in a hygienic material. The water-absorption rate of the water-absorbent resin is a value obtainable by a measurement method described in Examples set forth below.

In the water-absorbent sheet structure of the present invention, it is preferable that there is a positive difference in values between the water absorption rate of saline solution of a water-absorbent resin used in the primary absorbent layer and the rate of that used in the secondary absorbent layer. The greater the difference therebetween, effects of avoiding the stagnation of a liquid in the primary absorbent layer, to thereby increase dry feel, and effects of preventing a liquid leakage are even more strongly exhibited. Specifically, (the water absorption rate of saline solution of a water-absorbent resin used in the primary absorbent layer) - (the water absorption rate of saline solution of a water-absorbent resin used in the secondary absorbent layer) is preferably 10 seconds or more, more preferably 15 seconds or more, and even more preferably 20 seconds or more.

The water-absorbent resin has a mass-average particle size of preferably from 100 to 600 $\mu$m, more preferably from 150 to 550 $\mu$m, and even more preferably from 200 to 500 $\mu$m, from the viewpoint of preventing the scattering of the water-absorbent resin and the gel-blocking phenomenon during water absorption of the water-absorbent resin in the water-absorbent sheet structure, and at the same time reducing the rugged feel of the water-absorbent sheet structure, thereby improving texture.

The adhesive usable in the water-absorbent sheet structure according to the present invention includes, for example, rubber-based adhesives such as natural rubbers, butyl rubbers, and polyisoprene; styrene-based elastomer adhesives such as styrene-isoprene block copolymers (SIS), styrene-butadiene block copolymers (SBS), styrene-isobutylene block copolymers (SIBS), and styrene-ethylene-butylene-styrene block copolymers (SEBS); ethylene-vinyl acetate copolymer (EVA) adhesives; ethylene-acrylic acid derivative copolymer-based adhesives such as ethylene-ethyl acrylate copolymer (EEA), and ethylene-butyl acrylate copolymer (EBA); ethylene-acrylic acid copolymer (EAA) adhesives; polyamide-based adhesives such as copolymer nylons and dimer acid-based polyamides; polyolefin-based adhesives such as polyethylenes, polypropylenes, atactic polypropylenes, and copolymeric polyolefins; polyester-based adhesives such as polyethylene terephthalate (PET), polybutylene terephthalate (PBT), and copolymeric polyesters; and acrylic-based adhesives. In the present invention, the ethylene-vinyl acetate copolymer adhesives, the styrene-based elastomer adhesives, the polyolefin-based adhesives, and the polyester-based adhesives are preferred, from the viewpoint of high adhesive strength, thereby making it possible to prevent exfoliation of a nonwoven fabric and scattering of the water-absorbent resin in the water-absorbent sheet structure. These adhesives may be used alone, or they may be used in combination of two or more kinds.

When a thermal-fusing adhesive is used, the adhesive has a melting temperature or softening temperature of preferably from 60° to 180°C, and more preferably from 70° to 150°C, from the viewpoint of sufficiently fixing a water-absorbent resin to a nonwoven fabric, and at the same time preventing thermal deterioration or deformation of the nonwoven fabric.

The adhesive in the water-absorbent sheet structure is contained in a proportion in the range of from 0.05 to 2.0 times, preferably in the range of from 0.08 to 1.5 times, and more preferably in the range of from 0.1 to 1.0 time the amount of the water-absorbent resin contained (mass basis). The adhesive is contained in a proportion of 0.05 times or more, from the viewpoint of having sufficient adhesion, thereby preventing exfoliation of the nonwoven fabrics themselves or scattering of the water-absorbent resin, and increasing shape retaining ability of a water-absorbent sheet structure. The adhesive is contained in a proportion of 2.0 times or less, from the viewpoint of avoiding the inhibition of the swelling of the water-absorbent resin due to too strong adhesion to each other, thereby improving a permeation rate or liquid leakage of a water-absorbent sheet structure.

The present invention has a feature that an entire side or a part of the absorbent layer of the above-mentioned water-absorbent sheet structure has a structure in which the absorbent layer is separated in divided parts of a primary absorbent layer as an upper side and a secondary absorbent layer as a lower side with an appropriate fibrous substrate in a perpendicular direction, i.e. a thickness direction of the sheet.

As the above-mentioned fibrous substrate, those fibrous substrates having a void ratio of from 91 to 99%. Here, the void ratio of the fibrous substrate refers to a value calculated by the following formula.

Void Ratio of Fibrous Substrate (%)=[1-(M/(A x T x D))] x 100, wherein M is the mass (g) of the fibrous substrate, A is an area (cm$^2$) of the fibrous substrate, T is a thickness of the fibrous substrate, and D is a density (g/cm$^3$) of fibers forming the fibrous substrate.

It is necessary that the fibrous substrate is a material which appropriately permeates and appropriately diffuses a subject liquid in the substrate. Moreover, as the function of a fibrous substrate, if water permeability is regarded as important, gel blocking occurs, so that it is necessary to find a material with a proper balance to be used as a water-absorbent sheet structure. In addition, in a case where the water-absorbent sheet structure according to the present invention is used for a water-absorbent article such as disposable diapers, the above-mentioned water-absorbent sheet structure absorbs a body fluid in a state that a load from human body, i.e. body weight, is applied. In the state that a load is applied to the water-absorbent sheet structure as described above, a liquid permeation rate is likely to be large. Further, this tendency becomes greater as the thickness of the water-absorbent sheet structure becomes thinner, and there would pose limitations in thinning of the water-absorbent sheet structure if such phenomena are to be avoided.

The present inventors have found that a water-absorbent sheet structure having an excellent liquid permeation rate under load is obtained even when the water-absorbent sheet structure is being thin, by using a fibrous substrate having a specified void ratio, and the present invention is accomplished thereby. The above-mentioned fibrous substrate has a void ratio within the range of from 91 to 99%, preferably within the range of from 92 to 98%, and more preferably within the range of from 93 to 97%. The fibrous substrate has a void ratio of 91% or more, from the viewpoint of suppressing excessive diffusion of the absorbed liquid, and from the viewpoint of allowing the fibrous substrate to have an appropriate void even under load, and fast liquid permeation rate. The fibrous substrate has a void ratio of 99% or less, from the viewpoint of suppressing excessive water permeation of the absorbed liquid, and from the viewpoint of obtaining sufficient strength against stretching and twisting during the production and upon use of a water-absorbent sheet structure.

Upon the selection of the material for the above-mentioned fibrous substrate, when a fiber of which liquid diffusion is in excess is selected, a secondary absorbent layer cannot be effectively used, so that there is a risk of causing a liquid leakage. On the other hand, when a fiber of which water permeation of the liquid is in excess is selected, a primary absorbent layer cannot be effectively used, so that liquid is rapidly distributed in the secondary absorbent layer, thereby having a risk of causing gel blocking. From the viewpoints of liquid diffusion, liquid permeation mentioned above and the like, the fibrous substrate has a degree of hydrophilicity of preferably from 5 to 200, more preferably from 8 to 150, even more preferably from 10 to 100, and still even more preferably from 12 to 80, when the degree of hydrophilicity is measured in accordance with the method for measuring "Degree of Hydrophilicity of Nonwoven Fabric" described later. Preferred specific examples of the fibrous substrate include sanitary papers, nonwoven fabrics made of cellulose-containing synthetic fibers, nonwoven fabrics made of rayon-containing synthetic fibers, nonwoven fabrics made of hydrophilically treated synthetic fibers, and the like.

The sanitary papers include, for examples, tissue paper, water-absorbing paper, paper towel, and the like. The above-mentioned nonwoven fabrics made of cellulose-containing synthetic fibers include, for example, airlaid nonwoven fabrics made of pulp/PET/polyethylene (PE), pulp/PET/polypropylene (PP), or pulp/PE/PP. The above-mentioned nonwoven fabrics made of rayon-containing synthetic fibers include, for example, spunlace nonwoven fabrics made of rayon/PET, rayon/PE, or rayon/PET/PE. The above-mentioned nonwoven fabrics made of hydrophilically treated synthetic fibers include, for example, an air-through nonwoven fabric made of a polyolefin comprising PE, PP, or PE/PP coated with a hydrophilic surfactant such as a fatty acid ester-type nonionic surfactant or a polyglycerol fatty acid ester. Among these fibrous substrates, the nonwoven fabrics made of rayon-containing synthetic fibers and the nonwoven fabrics made of hydrophilically treated synthetic fibers are preferably used, and the nonwoven fabrics made of hydrophilically treated synthetic fibers are more preferably used, from the viewpoint of the void ratio and the strength of the fibrous substrate and various properties (liquid re-wet, shape retaining ability and the like) of the resulting water-absorbent sheet structure. Here, these fibrous substrates may be nonwoven fabrics made of single fibers, or nonwoven fabrics made of two or more kinds of fibers used in combination.

The thickness and the basis weight of the above-mentioned fibrous substrate are not particularly limited, so long as they are within the range that would satisfy the void ratio mentioned above. Exemplifying more preferred forms, the fibrous substrate has a thickness of preferably 200 $\mu$m or more, more preferably from 250 to 2,000 $\mu$m, and even more preferably from 300 to 1,000 $\mu$m. In addition, the fibrous substrate has a basis weight of preferably 15 g/m$^2$ or more, and more preferably from 20 to 200 g/m$^2$. It is preferable that the fibrous substrate has a thickness of 2,000 $\mu$m or less, and that the fibrous substrate has a basis weight of 200 g/m$^2$ or less, from the viewpoint of thinning a water-absorbent sheet structure. On the other hand, it is preferable that the fibrous substrate has a thickness of 200 $\mu$m or more, and that the fibrous substrate has a basis weight of 15 g/m$^2$ or more, from the viewpoint of obtaining sufficient strength against stretching and twisting during the production and upon use of a water-absorbent sheet structure, and from the viewpoint of improving various properties of the water-absorbent sheet structure under load. As the method for measurement of the thickness of the fibrous substrate, the method for measurement of the thickness of the water-absorbent sheet structure in a dry state described later can be employed.

The nonwoven fabrics usable in the water-absorbent sheet structure according to the present invention are not particularly limited, as long as the nonwoven fabrics are known nonwoven fabrics in the field of art. The nonwoven fabrics include nonwoven fabrics made of polyolefin fibers such as polyethylene (PE) and polypropylene (PP); polyester fibers such as polyethylene terephthalate (PET), polytrimethylene terephthalate (PTT), and polyethylene naphthalate (PEN); polyamide fibers such as nylon; rayon fibers, and other synthetic fibers; nonwoven fabrics produced by mixing cotton, silk, hemp, pulp (cellulose) fibers, or the like, from the viewpoint of liquid permeability, flexibility and shape retaining ability upon forming into a water-absorbent sheet structure. Among these nonwoven fabrics, the nonwoven fabrics made of synthetic fibers are preferably used, from the viewpoint of increasing the shape retaining ability of the water-absorbent sheet structure. Especially, nonwoven fabrics made of rayon fibers, polyolefin fibers, or polyester fibers are preferred. These nonwoven fabrics may be nonwoven fabrics made of single fibers mentioned above, or nonwoven fabrics made of two or more kinds of fibers used in combination.

More specifically, spunbond nonwoven fabrics made of fibers selected from the group consisting of polyolefin fibers, polyester fibers and blends thereof are more preferred, from the viewpoint of increasing shape retaining ability of the water-absorbent sheet structure, and preventing pass of the water-absorbent resin through the nonwoven fabric. In addition, spunlace nonwoven fabrics made of rayon fibers as a main component and air-through nonwoven fabrics made of polyolefin fibers are also more preferred as the nonwoven fabrics used in the present invention, from the viewpoint of even more increasing liquid absorbent properties and flexibility upon formation of the sheet. Among the spunbond nonwoven fabrics mentioned above, spunbond-meltblown-spunbond (SMS) nonwoven fabrics and spunbond-meltblown-meltblown-spunbond (SMMS) nonwoven fabrics, which have a multi-layered structure of polyolefin fibers are more preferably used, and the SMS nonwoven fabrics and the SMMS nonwoven fabrics each made of polypropylene fibers as a main component are especially preferably used. On the other hand, as the above-mentioned spunlace nonwoven fabrics, those of proper blends of main component rayon fibers with polyolefin fibers and/or polyester fibers are preferably used, and among them, rayon-PET nonwoven fabrics and rayon-PET-PE nonwoven fabrics are preferably used. The above-mentioned nonwoven fabrics may contain a small amount of pulp fibers to an extent that would not increase the thickness of the water-absorbent sheet structure.

When the hydrophilicity of the above-mentioned nonwoven fabric is too low, the liquid absorbent properties of the water-absorbent sheet structure is worsened, and on the other hand, when the hydrophilicity is much higher than a necessary level, the liquid absorbent properties would not be improved to the level equivalent thereto. Therefore, it is desired that the nonwoven fabric has an appropriate level of hydrophilicity. From those viewpoints, the nonwoven fabrics having a degree of hydrophilicity of from 5 to 200 are preferably used, more preferably those having a degree of hydrophilicity of from 8 to 150, even more preferably those having a degree of hydrophilicity of from 10 to 100, and still even more preferably those having a degree of hydrophilicity of from 12 to 80 when measured in accordance with the method for measuring "Degree of Hydrophilicity of Nonwoven Fabric" described later. The nonwoven fabric having hydrophilicity as mentioned is not particularly limited, and among the nonwoven fabrics mentioned above, those of which materials themselves show hydrophilicity such as rayon fibers may be used, or those obtained by subjecting hydrophobic chemical fibers such as polyolefin fibers or polyester fibers to a hydrophilic treatment according to a known method to give an appropriate degree of hydrophilicity may be used. The method of hydrophilic treatment includes, for example, a method including subjecting, in a spunbond nonwoven fabric, a mixture of a hydrophobic chemical fiber with a hydrophilic treatment agent to a spunbond method to give a nonwoven fabric; a method including carrying a hydrophilic treatment agent along upon the preparation of a spunbond nonwoven fabric with a hydrophobic chemical fiber; a method including obtaining a spunbond nonwoven fabric with a hydrophobic chemical fiber, and thereafter impregnating the nonwoven fabric with a hydrophilic treatment agent, or the like. As the hydrophilic treatment agent, an anionic surfactant such as an aliphatic sulfonic acid salt or a sulfuric acid ester of a higher alcohol; a cationic surfactant such as a quaternary ammonium salt; a nonionic surfactant such as a polyethylene glycol fatty acid ester, a polyglycerol fatty acid ester, or a sorbitan fatty acid ester; a silicone-based surfactant such as a polyoxyalkylene-modified silicone; a stain-release agent made of a polyester-based, polyamide-based, acrylic, or urethane-based resin; or the like is used.

It is preferable that the nonwoven fabrics that sandwich the absorbent layer is hydrophilic, from the viewpoint of even more increasing the liquid absorbent properties of the water-absorbent sheet structure. Especially from the viewpoint of preventing liquid leakage, it is more preferable that hydrophilic property of a nonwoven fabric used in a lower side of an absorbent layer is equivalent to or higher than hydrophilic property of a nonwoven fabric used in an upper side of the absorbent layer. The upper side of an absorbent layer as used herein refers to a side to which a liquid to be absorbed is supplied at the time of preparing an absorbent article using the water-absorbent sheet structure obtained, and the lower side of an absorbent layer refers to a side opposite thereof.

The nonwoven fabric is preferably a nonwoven fabric having an appropriate bulkiness and a large basis weight, from the viewpoint of giving the water-absorbent sheet structure according to the present invention excellent liquid permeability, flexibility, shape retaining ability and cushioning property, and speeding up the liquid permeation rate of the water-absorbent sheet structure. The nonwoven fabric has a basis weight of preferably from 5 to 300 $g/m^2$, more preferably from 8 to 200 $g/m^2$, even more preferably from 10 to 100 $g/m^2$, and still even more preferably from 11 to 50 $g/m^2$. Also, the nonwoven fabric has a thickness of preferably in the range of from 20 to 800 $\mu$m, more preferably in the range of from 50 to 600 $\mu$m, and even more preferably in the range of from 80 to 450 $\mu$m. As the method for measurement of the thickness of the nonwoven fabric, the method for measurement of the thickness of the water-absorbent sheet structure in a dry state described later can be employed.

The water-absorbent sheet structure according to the present invention can be produced by a method, for example, as described in a method as described hereinbelow.

(a) A mixed powder of a water-absorbent resin and an adhesive is evenly dispersed over a nonwoven fabric, a fibrous substrate is overlaid thereto, and the overlaid layers are subjected to pressing while heating near a melting temperature of the adhesive to obtain an intermediate product. The mixed powder is again dispersed over this intermediate product in the same manner as above, a nonwoven fabric is further overlaid thereto, and the overlaid layers are subjected to pressing while heating.

(b) A mixed powder of a water-absorbent resin and an adhesive is evenly dispersed over a nonwoven fabric, a fibrous substrate is overlaid thereto, thereafter the mixed powder is again dispersed, a nonwoven fabric is further overlaid thereto, and the overlaid layers are together subjected to pressing while heating.

(c) A mixed powder of a water-absorbent resin and an adhesive is evenly dispersed over a nonwoven fabric, and passed through a heating furnace to fix the powder to an extent that the powder does not scatter. A fibrous substrate is overlaid thereto, the mixed powder is again dispersed, a nonwoven fabric is overlaid thereto, and the overlaid layers are together subjected to pressing while heating.

(d) An adhesive is melt-coated over a nonwoven fabric, a water-absorbent resin is immediately thereafter evenly dispersed thereto to form a layer, further an adhesive is melt-coated from an upper side, and a fibrous substrate is overlaid thereto, to obtain an intermediate product. Immediately after an adhesive is melt-coated in the same manner as above over this intermediate product, a water-absorbent resin is evenly dispersed thereto to form a layer, further an adhesive is melt-coated from an upper side, a fibrous substrate is overlaid thereto, and the overlaid layers are together subjected to pressing while heating.

Here, among the methods exemplified in (a) to (d), the water-absorbent sheet structure can be produced by separately selecting methods for adhering a primary absorbent layer and a secondary absorbent layer, and then combining the methods. The water-absorbent sheet structure may be subjected to emboss treatment during the pressing while heating in the production of a water-absorbent sheet structure or after the production of the water-absorbent sheet structure, for the purposes of improving the feel and improving shape retaining ability of the water-absorbent sheet structure.

In addition, the water-absorbent sheet structure according to the present invention may properly be formulated with an additive such as a deodorant, an anti-bacterial agent, or a gel stabilizer.

The water-absorbent sheet structure according to the present invention has one feature in the point of enabling thinning of the sheet. When the use in absorbent articles is taken into consideration, the water-absorbent sheet structure has a thickness, in a dry state, of preferably 4 mm or less, more preferably from 3 mm or less, and even more preferably from 1.0 to 2.5 mm. The dry state refers to a state before which the water-absorbent sheet structure absorbs a liquid. In the present specification, the thickness of the water-absorbent sheet structure in a dry state is a value obtainable by a measurement method described in Examples set forth below.

Further, the water-absorbent sheet structure according to the present invention has one feature in that the water-absorbent sheet structure has a fast liquid permeation rate under load, and the water-absorbent sheet structure has a liquid permeation rate under load of preferably 1,000 seconds or less, more preferably 800 seconds or less, and even more preferably 700 seconds or less, when its use in an absorbent article is taken into consideration. In the present specification, the liquid permeation rate under load of the water-absorbent sheet structure is a value obtainable by a measurement method described in Examples set forth below.

Further, the water-absorbent sheet structure according to the present invention has one feature in that the water-absorbent sheet structure has smaller slope liquid leakage, and the water-absorbent sheet structure has a leakage index of preferably 200 or less, more preferably 100 or less, and even more preferably 50 or less, when its use in an absorbent article is taken into consideration. In the present specification, the slope leakage index of the water-absorbent sheet structure is a value obtainable by a measurement method described in Examples set forth below.

As the water-absorbent sheet structure according to the present invention, those having given properties of a thickness in a dry state, a liquid permeation rate under load, and a leakage index are preferred.

Further, since the water-absorbent sheet structure according to the present invention has a very small amount of a material derived from nature, consideration has been made to the environment while having high performance in thickness, permeation rate, and a leakage index as mentioned above. The proportion of the natural material used is preferably 30% by mass, more preferably 20% by mass or less, and even more preferably 15% or less. The proportion of the natural material used is calculated by dividing a total content of pulp, cotton, hemp, silk, and the like contained in very small amounts in each of the constituents of the water-absorbent sheet structure by mass of the water-absorbent sheet structure.

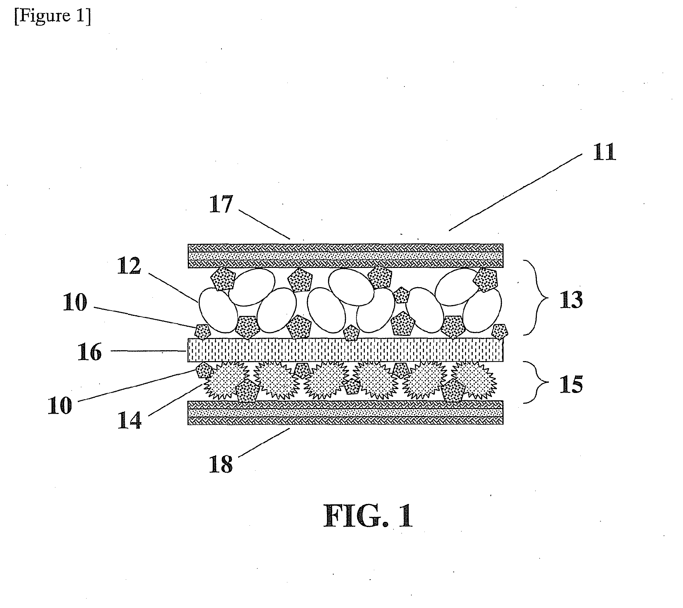

Next, the structure of the water-absorbent sheet structure of the present invention will be explained by referring to Figure 1. Here, Figure 1 is an enlarged cross-sectional view schematically showing a structure of a water-absorbent sheet structure of the present invention.

A water-absorbent sheet structure 11 shown in Figure 1 comprises a primary absorbent layer 13 containing a water-absorbent resin 12 and an adhesive 10, and a secondary absorbent layer 15 containing a water-absorbent resin 14 and an adhesive 10. Here, the primary absorbent layer refers to a side to which a liquid to be absorbed is supplied upon the preparation of an absorbent article using the water-absorbent sheet structure, and the secondary absorbent layer refers to a side opposite to primary absorbent layer sandwiched with a fibrous substrate 16.

Moreover, the primary absorbent layer 13 and the secondary absorbent layer 15 are separated in divided parts with a fibrous substrate 16, and a water-absorbent sheet structure 11 has a five-layer structure, comprising a primary absorbent layer 13, a secondary absorbent layer 15, a fibrous substrate 16, and two layers, front and back, of nonwoven fabrics 17 and 18 positioned on each of outer sides of the primary absorbent layer 13 and the secondary absorbent layer 15. The absorbent layers have a structure that the absorbent layers are sandwiched with the nonwoven fabrics 17 and 18 from an upper side and a lower side.

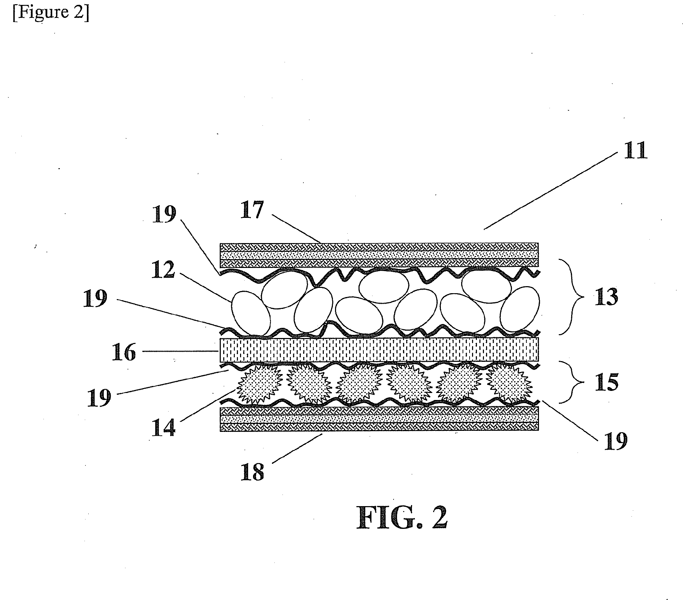

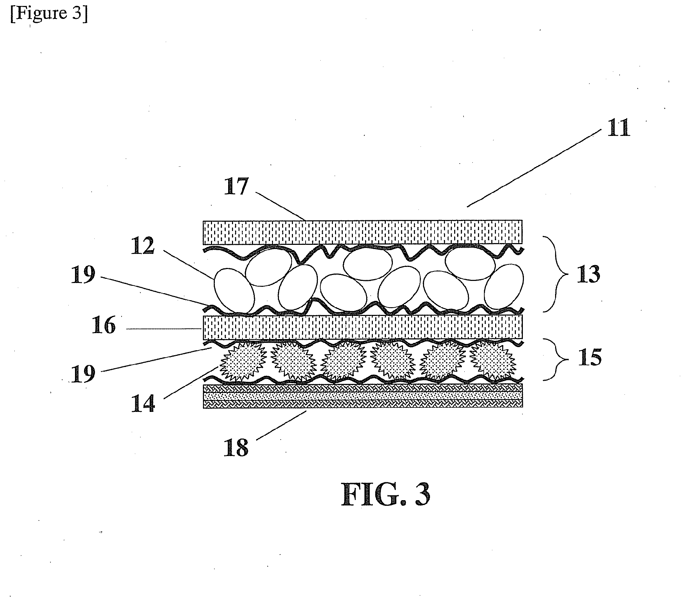

In addition, the water-absorbent sheet structures shown in Figures 2 and 3 are also exemplifications of other embodiments of the water-absorbent sheet structure of the present invention. Figure 2 is an example where an adhesive 19 is melt-coated on a nonwoven fabric 17 or the like. Figure 3 is an example where in the nonwoven fabric 17 in Figure 1, the same material as in the fibrous substrate (nonwoven fabric) 16 is used.

The absorbent article according to the present invention can be obtained by sandwiching a water-absorbent sheet structure according to the present invention between a liquid-permeable sheet and a liquid-impermeable sheet. As the liquid-permeable sheet and the liquid-impermeable sheet, known ones in the technical field of the absorbent articles can be used without particular limitations. Also, the absorbent article can be produced by a known method.

The above-mentioned absorbent article includes, for example, disposable diapers, incontinence pads, sanitary napkins, pet sheets, drip sheets for foods, water blocking materials for electric power cables, and the like.

**EXAMPLES**

The present invention will be specifically described hereinbelow by the Examples, without intending to limit the scope of the present invention thereto.

The properties of the water-absorbent resin and the water-absorbent sheet structure were measured and evaluated in accordance with the following methods.

< Water-Retention Capacity of Saline Solution of Water-Absorbent Resin >

The amount 2.0 g of water-absorbent resin was weighed in a cotton bag (Cottonbroad No. 60, width 100 mm x length 200 mm), and placed in a 500 mL-beaker. Saline solution (0.9% by mass aqueous solution of sodium chloride, hereinafter referred to the same) was poured into the cotton bag in an amount of 500 g at one time, and the saline solution was dispersed so as not to cause an unswollen lump of the water-absorbent resin. The upper part of the cotton bag was tied up with a rubber band, and the cotton bag was allowed to stand for 1 hour, to sufficiently make the water-absorbent resin swollen. The cotton bag was dehydrated for 1 minute with a dehydrator (manufactured by Kokusan Enshinki Co., Ltd., product number: H-122) set to have a centrifugal force of 167G. The mass Wa (g) of the cotton bag containing swollen gels after the dehydration was measured. The same procedures were carried out without adding water-absorbent resin, and the empty mass Wb (g) of the cotton bag upon wetting was measured. The water-retention capacity of saline solution of the water-absorbent resin was calculated from the following formula.

$$\text{Water-Retention Capacity of Saline Solution (g/g) of}$$

$$\text{Water-Absorbent Resin}$$

$$= [Wa - Wb] \text{ (g)/Mass (g) of Water-Absorbent Resin}$$

< Water-Absorption Rate of Saline Solution of Water-Absorbent Resin >

This test was conducted indoors temperature-controlled to $25° \pm 1°C$. The amount $50 \pm 0.1$ g of saline solution was weighed out in a 100 mL beaker, and a magnetic stirrer bar (8 mm$\phi$ x 30 mm, without a ring) was placed therein. The beaker was immersed in a thermostat, of which liquid temperature was controlled to $25° \pm 0.2°C$. Next, the beaker was placed over the magnetic stirrer so that a vortex was generated in saline solution at a rotational speed of 600 r/min, the water-absorbent resin was then quickly added in an amount of $2.0 \pm 0.002$ g to the above beaker, and the time period (seconds) from a point of addition of the water-absorbent resin to a point of convergence of the vortex of the liquid surface was measured with a stopwatch, which was defined as a water-absorption rate of the water-absorbent resin.

< Mass-Average Particle Size of Water-Absorbent Resin >

An amorphous silica (Sipernat 200, Degussa Japan) was mixed in an amount of 0.5 g as a lubricant with 100 g of a water-absorbent resin, to prepare a water-absorbent resin for measurement.
The above-mentioned water-absorbent resin was allowed to pass though a JIS standard sieve having a sieve opening of 250 $\mu$m, and a mass-average particle size was measured using a combination of sieves of (A) in a case where the resin was allowed to pass in an amount of 50% by mass or more, or a combination of sieves of (B) in a case where the resin was allowed to pass in an amount of less than 50% by mass.

(A) JIS standard sieves, a sieve having an opening of 425 $\mu$m, a sieve having an opening of 250 $\mu$m, a sieve having an opening of 180 $\mu$m, a sieve having an opening of 150 $\mu$m, a sieve having an opening of 106 $\mu$m, a sieve having an opening of 75 $\mu$m, a sieve having an opening of 45 $\mu$m, and a receiving tray were combined in order from the top.

(B) JIS standard sieves, a sieve having an opening of 850 $\mu$m, a sieve having an opening of 600 $\mu$m, a sieve having an opening of 500 $\mu$m, a sieve having an opening of 425 $\mu$m, a sieve having an opening of 300 $\mu$m, a sieve having an opening of 250 $\mu$m, a sieve having an opening of 150 $\mu$m, and a receiving tray were combined in order from the top.

The above-mentioned water-absorbent resin was placed on an uppermost sieve of the combined sieves, and shaken for 20 minutes with a rotating and tapping shaker machine to classify the resin.

After classification, the relationships between the opening of the sieve and an integral of a mass percentage of the water-absorbent resin remaining on the sieve were plotted on a logarithmic probability paper by calculating the mass of the water-absorbent resin remaining on each sieve as a mass percentage to an entire amount, and accumulating the mass percentages in order, starting from those having larger particle diameters. A particle diameter corresponding to a 50% by mass cumulative mass percentage is defined as a mass-average particle size by joining the plots on the probability paper in a straight line.

< Degree of Hydrophilicity of Nonwoven Fabric >

In the present specification, the degree of hydrophilicity of the nonwoven fabrics was measured using an apparatus described in "Determination of Water Repellency" described in JAPAN TAPPI Test Method No. 68 (2000).

Specifically, a nonwoven fabric test piece, which was cut into a rectangular strip having width x length dimensions of 10 cm x 30 cm in a manner so that the longitudinal direction to be in a length direction (machine feeding direction) of the nonwoven fabric, was attached to a test piece attaching apparatus sloped at 45°. A burette controlled at an opening of a cock of the burette so that 10 g of distilled water was supplied in 30 seconds was once dried, and fixed so that a part 5 mm above in a vertical direction from the uppermost part of a test piece attached to the sloped apparatus was arranged at the tip of the burette. About 60 g of distilled water was supplied from the upper part of the burette, and a time period (seconds) from the beginning of dripping of a liquid to a nonwoven fabric test piece from the tip of the burette to a point where the liquid leaked out from a lower part because the test piece could not hold on to the liquid was measured, and defined as a degree of hydrophilicity of a nonwoven fabric. It is judged that the larger the numerical values, the higher the degree of hydrophilicity.

Usually, the material itself of the nonwoven fabric having hydrophilicity or a nonwoven fabric provided with a hydrophilic treatment has a numerical value for a degree of hydrophilicity of 5 or more, while in a nonwoven fabric of a material having a low hydrophilicity, liquids are more likely to run over near the surface and leak out from a lower part more quickly.

< Measurement of Thickness of Water-Absorbent Sheet Structure in Dry State >

A water-absorbent sheet structure, which was cut into rectangular strips having dimensions of 19 cm x 51 cm in a manner that a longitudinal direction thereof is to be in a length direction (machine feeding direction) of the nonwoven fabric, was used as a sample. The thickness of the resulting water-absorbent sheet structure was measured using a thickness measurement instrument (manufactured by Kabushiki Kaisha Ozaki Seisakusho, model number: J-B) at three measurement sites taken in a longitudinal direction, on the left end, the center, and the right end; for example, the left end was set at a site 5 cm away from the left side, the center was set at a site 25.5 cm away therefrom, and the right end was set at a site 45 cm away therefrom. As the width direction, a central part was measured. The measurement value for thickness was obtained by measuring three times at each site, and an average for each site was obtained. Further, the values at the left end, the center, and the right end were averaged, which was defined as a thickness of an overall water-absorbent sheet structure in a dry state.

< Shape Retaining Ability of Water-Absorbent Sheet Structure >

The shape retaining ability of the water-absorbent sheet structure was evaluated in accordance with the following method.

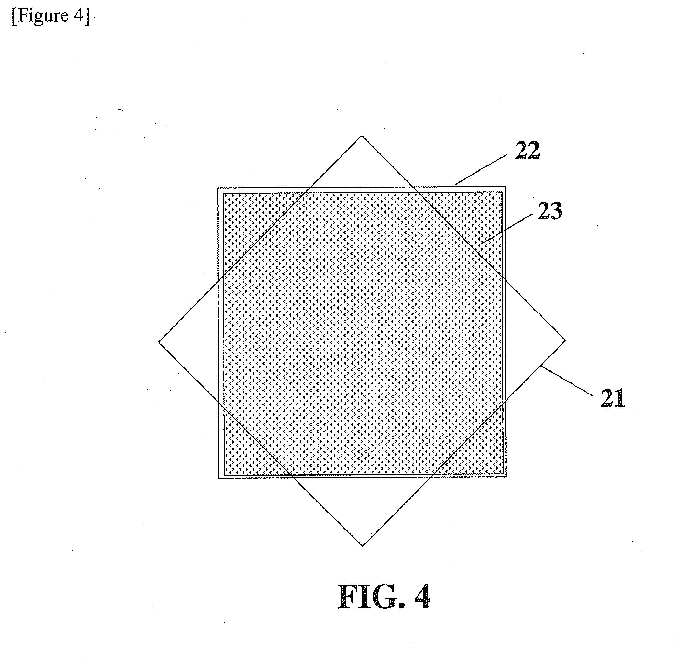

The resulting water-absorbent sheet structure was cut into a size of 10 cm x 10 cm. Next, the entire side of each of one side of two pieces of acrylic plates of 10 cm x 10 cm (mass: about 60 g) was adhered with a double-sided adhesive tape. As shown in Figure 4, an acrylic plate 21 was adhered to a water-absorbent sheet structure 23 to fix with pressure in a manner that the diagonal lines of acrylic plates 21, 22 formed 45 degrees in angle, and that the double-sided adhesive tape faced the side of the water-absorbent sheet structure 23.

The test pieces of the water-absorbent sheet structure prepared in the manner as described above were placed on a metallic tray of sieves, used in the section of the above-mentioned < Mass-Average Particle Size of Water-Absorbent Resin >, and a lid was put thereon. Thereafter, the lidded metallic tray was tapped with rotations with a rotating and tapping shaker machine for 3 minutes. The shape retaining ability of the water-absorbent sheet structure was evaluated based on the external appearance of the test pieces after tapping in accordance with the following criteria.

A: The water-absorbent sheet structure showed no changes in external appearance, and did not easily move even when the acrylic plates were tried to be displaced.
B: The water-absorbent sheet structure showed no changes in external appearance, but the water-absorbent sheet structure was split when the acrylic plates were displaced.
C: The water-absorbent sheet structure was split, and the contents were scattered.

< Evaluations of Liquid Permeation Rate Under Load and Amount of Re-wet of Water-Absorbent Sheet Structure >

A water-absorbent sheet structure, which was cut into rectangular strips having dimensions of 19 cm x 51 cm in a manner that a longitudinal direction thereof is to be in a length direction (machine feeding direction) of the nonwoven fabric, was used as a sample.

In a 10 L container were placed 60 g of sodium chloride, 1.8 g of calcium chloride dihydrate, 3.6 g of magnesium chloride hexahydrate, and a proper amount of distilled water to completely dissolve. Next, 15 g of an aqueous 1% by mass poly(oxyethylene) isooctylphenyl ether solution was added thereto, and distilled water was further added to adjust the weight of the overall aqueous solution to 6,000 g. Thereafter, the mixed solution was colored with a small amount of Blue No. 1 to prepare a test solution.

A polyethylene air-through style porous liquid-permeable sheet having the same size as the sample (19 cm x 51 cm) and a basis weight of 22 $g/m^2$ was placed over an upper side of a sample (water-absorbent sheet structure). In addition, underneath the sample was placed a polyethylene liquid-impermeable sheet having the same size and basis weight as the sheet, to prepare a simple absorbent article. An acrylic plate having a cylindrical cylinder with an inner diameter of 4.3 cm and of a height 12 cm was placed near the central section of this absorbent article, and further the weight was placed on the acrylic plate so as to apply a load of 3120 g in total to the sample. A 150 mL test solution was supplied thereto at one time. At the same time, a time period until the test solution was completely permeated into the absorbent article was measured with a stopwatch, which is referred to as a first permeation rate (seconds). Next, the same procedures were carried out placing the cylindrical cylinder at the same position as the first permeation rate 15 minutes thereafter, to measure second permeation rate (seconds). A total of the number of seconds for the first and second permeation rates is referred to as a liquid permeation rate under load.

After 30 minutes from the start of the feeding of the first test solution, the above-mentioned acrylic plate was removed, filter papers (about 80 sheets) of 10 cm each side, of which mass (Wc (g), about 70 g) was previously measured, were stacked near the liquid supplying position of the absorbent article, and a 5 kg weight of which bottom side has dimensions of 10 cm x 10 cm was placed thereon. After 5 minutes of applying a load, the mass (Wd (g)) of the filter papers was measured, and an increased mass was defined as the amount of re-wet (g).

$$\text{Amount of Re-wet (g)} = \text{Wd - Wc}$$

< Slope Leakage Test >

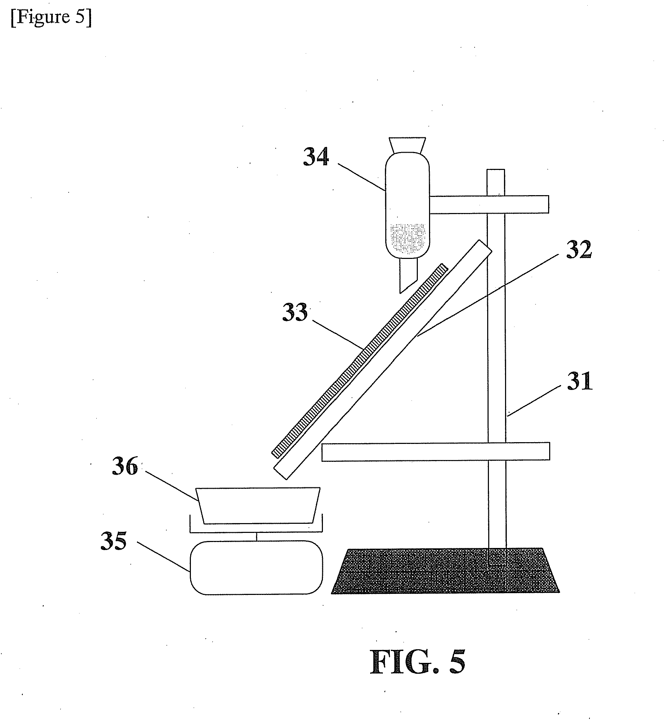

A slope leakage test was conducted using an apparatus shown in Figure 5.
Schematically, a mechanism is as follows. A commercially available stand 31 for experimental facilities was used to slope an acrylic plate 32 and fixed, the above-mentioned test solution was then supplied to an absorbent article 33 placed on the plate from a dropping funnel 34 positioned vertically above the absorbent article, and a leakage amount was measured with a balance 35. The detailed specifications are given hereinbelow.

An acrylic plate 32 has a length in the direction of the slope plane of 70 cm, and fixed so that an angle formed with a stand 31 against the horizontal is 45° ± 2°. The acrylic plate 32 had a width of 100 cm and a thickness of 1 cm, and plural absorbent articles 33 could be concurrently measured. The acrylic plate 32 had a smooth surface, so that a liquid was not detained or absorbed to the plate.

A dropping funnel 34 was fixed at a position vertically above the sloped acrylic plate 32 using the stand 31. The dropping funnel 34 had a volume of 100 mL, and an inner diameter of a tip end portion of about 4 mm, and an aperture of the cock was adjusted so that a liquid was supplied at a rate of 8 mL/s.

A balance 35 on which a tray 36 was placed was set at a lower side of the acrylic plate 32, and all the test solutions flowing down the plate were received as leakage, and the mass was recorded to the accuracy of 0.1 g.

A slope leakage test using an apparatus as described above was carried out in accordance with the following procedures. The mass of a water-absorbent sheet structure cut into a rectangular strip having dimensions of length 51 cm x width 19 cm in a manner that the longitudinal direction is a length direction (machine feeding direction) of the nonwoven fabric was measured, and an air-through style polyethylene liquid-permeable nonwoven fabric (basis weight: 22 g/m$^2$) of the same size was attached from an upper side thereof, and further a polyethylene liquid-impermeable nonwoven fabric having the same size and the same basis weight was attached from a lower side thereof to prepare a simple absorbent article 33. The simple absorbent article 33 was adhered on the acrylic plate 32 (in order not to stop leakage intentionally, the bottom end of the absorbent article 33 was not adhered to the acrylic plate 32).

Marking was put on the absorbent article 33 at a position 3 cm away in a downward direction from a top end thereof, and a supplying inlet for the dropping funnel 34 was fixed so that the inlet was positioned at a distance 8 mm ± 2 mm vertically above the marking.

A balance 35 was turned on, and tared so that the indication was zero, and thereafter 150 mL of the above-mentioned test solution was supplied at one time to the dropping funnel 34. An amount of liquid poured into a tray 36 after the test solution was allowed to flow over a sloped acrylic plate 32 without being absorbed into an absorbent article 33 was measured, and this amount of liquid was defined as a first leakage amount (g). The numerical value for this first leakage amount (g) was denoted as LW1.

Second test solution was supplied in 10 minutes after the beginning of the first supply, and second leakage amount (g) were measured, and the numerical value therefor was denoted as LW2.

Next, a leakage index was calculated in accordance with the following equation. The smaller the index, the smaller the leakage amount at a slope of a water-absorbent sheet structure, especially an initial leakage amount, whereby it is judged to be an excellent water-absorbent sheet structure.

$$\text{Leakage Index:} \quad L = LW1 \times 10 + LW2$$

(Example 1)

A roller spreader (manufactured by HASHIMA CO., LTD., SINTERACE M/C) was charged at its supplying inlet with a mixture prepared by homogeneously mixing 40 parts by mass of an ethylene-vinyl acetate copolymer (EVA; melting point: 95°C) as an adhesive and 200 parts by mass of a sodium polyacrylate crosslinked product (manufactured by Sumitomo Seika Co., Ltd., AQUAKEEP SA55SX-II, mass average particle size: 360 μm; water-absorption rate of saline solution: 42 seconds, water-retention capacity of saline solution: 35 g/g, referred to as "a water-absorbent resin A") as a water-absorbent resin. On the other hand, a spunbond-meltblown-spunbond (SMS) nonwoven fabric having a width of 30 cm hydrophilically treated with a hydrophilic treatment agent (fiber: polypropylene, void ratio: 90%, basis weight: 13 g/m$^2$, thickness: 150 μm, degree of hydrophilicity: 16, referred to as "Nonwoven Fabric A") was spread over a conveyor at the bottom part of the roller spreader. Next, the spreading roller and the bottom part conveyor were operated, thereby allowing the above-mentioned mixture to evenly overlay the above-mentioned nonwoven fabric at a basis weight of 240 g/m$^2$.

The overlaid product obtained was sandwiched from the top side with a water-absorbent paper (fiber: pulp, void ratio: 95%, basis weight: 25 g/m$^2$, thickness: 350 μm, degree of hydrophilicity: 55) as a fibrous substrate, and thereafter heat-fused with a laminating machine (manufactured by HASHIMA CO., LTD., straight linear fusing press HP-600LF) of which heating temperature was set at 130°C to integrate, to give an intermediate product of a water-absorbent sheet structure.

Next, a roller spreader was charged at its supplying inlet with a mixture prepared by homogeneously mixing 18 parts by mass of the above-mentioned EVA as an adhesive and 50 parts by mass of a sodium polyacrylate crosslinked product (manufactured by Sumitomo Seika Co., Ltd., AQUAKEEP 10SH-PB, mass-average particle size: 320 μm; water-absorption rate of saline solution: 3 seconds, water-retention capacity of saline solution: 42 g/g, referred to as "a water-absorbent resin B") as a water-absorbent resin. On the other hand, the intermediate product of a water-absorbent sheet structure was spread such that the side of the water-absorbent paper was placed on an upper side over the conveyor of the roller spreader. The spreading roller and the bottom part conveyor were operated, thereby allowing the above-mentioned mixture to evenly overlay over the above-mentioned water-absorbent paper, overlaid over the intermediate product of a water-absorbent sheet structure at a basis weight of 68 g/m$^2$.

The overlaid product obtained was sandwiched from a top side with the above-mentioned Nonwoven Fabric A in the same manner as above, and thereafter heat-fused with a laminating machine (manufactured by HASHIMA CO., LTD., straight linear fusing press HP-600LF) of which heating temperature was set at 130°C to integrate, to give a water-absorbent sheet structure. The cross section of the resulting water-absorbent sheet structure, as schematically shown, had a structure as shown in Figure 1.

The resulting water-absorbent sheet structure was cut into a given size with an absorbent layer using a water-absorbent resin A on an upper side (primary absorbent layer) to perform the above-mentioned various measurements and evaluations. The results are shown in Table 3.

(Example 2)

The above-mentioned Nonwoven Fabric A having a width of 30 cm was spread over a hot melt applicator (manufactured by HALLYS Corporation, Marshall 150) of which heating temperature was set at 150°C, and thereafter a styrene-butadiene-styrene copolymer (SBS, softening point: 85°C) was coated as an adhesive over the nonwoven fabric at a basis weight of 15 g/m$^2$.

Next, a roller spreader (manufactured by HASHIMA CO., LTD., SINTERACE M/C) was charged at its supplying inlet with a water-absorbent resin A. On the other hand, the above-mentioned Nonwoven Fabric A coated with an adhesive was spread over a conveyor at the bottom side of the spreader, with an adhesive-coated side on an upper side. Subsequently, the spreading roller and the bottom side conveyor were operated, thereby allowing the water-absorbent resin A to evenly overlay over the nonwoven fabric at a basis weight of 200 g/m$^2$.

The overlaid product obtained was sandwiched from a top side with a fibrous substrate [spunlace nonwoven fabric (fiber: rayon/PET, void ratio: 92%, basis weight: 35 g/m$^2$, thickness: 300 μm, degree of hydrophilicity: 38, referred to as "Nonwoven Fabric B") coated with the above-mentioned SBS as an adhesive in the same manner as above at a basis weight of 15 g/m$^2$, and thereafter heat-fused with a laminating machine (manufactured by HASHIMA CO., LTD., straight linear fusing press HP-600LF) of which heating temperature was set at 100°C to integrate, to give an intermediate product of a water-absorbent sheet structure.

In the same manner as above, the resulting intermediate product of a water-absorbent sheet structure was spread over the hot melt applicator of which heating temperature was set at 150°C, with the side of Nonwoven Fabric B on an upper side, and the above-mentioned SBS was coated as an adhesive over the Nonwoven Fabric B of the intermediate product of a water-absorbent sheet structure at a basis weight of 10 g/m$^2$.

Next, the roller spreader was charged at its supplying inlet with a water-absorbent resin B. On the other hand, the intermediate product of a water-absorbent sheet structure was spread over a conveyor at the bottom side of the spreader, with an adhesive-coated side on an upper side. Subsequently, the spreading roller and the bottom side conveyor were operated, thereby allowing the water-absorbent resin B to evenly overlay over the Nonwoven Fabric B of the above-mentioned intermediate product of a water-absorbent sheet structure at a basis weight of 50 g/m$^2$.

The overlaid product obtained was sandwiched from a top side with another Nonwoven Fabric A coated with the above-mentioned SBS at a basis weight of 10 g/m$^2$ in the same manner as above, and thereafter heat-fused with a laminating machine (manufactured by HASHIMA CO., LTD., straight linear fusing press HP-600LF) of which heating temperature was set at 100°C to integrate, to give a water-absorbent sheet structure. The cross section of the resulting water-absorbent sheet structure, as schematically shown, had a structure as shown in Figure 2.

The resulting water-absorbent sheet structure was cut into a given size with an absorbent layer using a water-absorbent resin A on an upper side (primary absorbent layer) to perform the above-mentioned various measurements and evaluations. The results are shown in Table 3.

(Example 3)

The same procedures as in Example 2 were carried out except that a fibrous substrate to be used was changed to an air-through nonwoven fabric (fiber: polypropylene/polyethylene hydrophilically treated with a hydrophilic treatment agent, void ratio: 92%, thickness: 150 μm, basis weight: 23 g/m$^2$, degree of hydrophilicity: 33; referred to as "Nonwoven Fabric C") in Example 2, to give a water-absorbent sheet structure.

The resulting water-absorbent sheet structure was cut into a given size with an absorbent layer using a water-absorbent resin A on an upper side (primary absorbent layer) to perform the above-mentioned various measurements and evaluations. The results are shown in Table 3.

(Examples 4)

The same procedures as in Example 2 were carried out except that a fibrous substrate to be used was changed to an air-through nonwoven fabric (fiber: polypropylene/polyethylene hydrophilically treated with a hydrophilic treatment agent, void ratio: 97%, thickness: 820 $\mu$m, basis weight: 20 g/m$^2$, degree of hydrophilicity: 12; referred to as "Nonwoven Fabric D") in Example 2, to give a water-absorbent sheet structure.
The resulting water-absorbent sheet structure was cut into a given size with an absorbent layer using a water-absorbent resin A on an upper side (primary absorbent layer) to perform the above-mentioned various measurements and evaluations. The results are shown in Table 3.

(Example 5)

The same procedures as in Example 2 were carried out except that a nonwoven fabric to be used in the production of an intermediate product of a water-absorbent sheet structure was changed to Nonwoven Fabric C, and a fibrous substrate was changed to Nonwoven Fabric C in Example 2, to give a water-absorbent sheet structure. The cross section of the resulting water-absorbent sheet structure, as schematically shown, had a structure as shown in Figure 3.
The resulting water-absorbent sheet structure was cut into a given size with an absorbent layer using a water-absorbent resin A on an upper side (primary absorbent layer) to perform the above-mentioned various measurements and evaluations. The results are shown in Table 3.

(Example 6)

The same procedures as in Example 2 were carried out except that the nonwoven fabric and the fibrous substrate to be used were all changed to Nonwoven Fabric D in Example 2, to give a water-absorbent sheet structure.
The resulting water-absorbent sheet structure was cut into a given size with an absorbent layer using a water-absorbent resin A on an upper side (primary absorbent layer) to perform the above-mentioned various measurements and evaluations. The results are shown in Table 3.

(Examples 7 and 8)

The same procedures as in Example 3 were carried out except that the content of water-absorbent resins A and B and the adhesive to be used were changed to those listed in Table 2 in Example 3, to give each of water-absorbent sheet structures.
The resulting water-absorbent sheet structures obtained in Examples 7 and 8 were cut into a given size with an absorbent layer using a water-absorbent resin A on an upper side (primary absorbent layer) to perform the above-mentioned various measurements and evaluations. The results are shown in Table 3.

(Comparative Example 1)

The above-mentioned Nonwoven Fabric A having width of 30 cm was spread over a hot melt applicator (manufactured by HALLYS Corporation, Marshall 150) of which heating temperature was set at 150°C, and thereafter the above-mentioned SBS (softening point: 85°C) was coated as an adhesive over the nonwoven fabric at a basis weight of 25 g/m$^2$.
Next, a roller spreader (manufactured by HASHIMA CO., LTD., SINTERACE M/C) was charged at its supplying inlet with a water-absorbent resin A. On the other hand, the above-mentioned Nonwoven Fabric A coated with the adhesive was spread over a conveyor of the roller spreader, with an adhesive-coated side on an upper side. Subsequently, the spreading roller and the bottom part conveyor were operated, thereby allowing the water-absorbent resin A to evenly overlay the nonwoven fabric at a basis weight of 250 g/m$^2$.
The overlaid product obtained was sandwiched from a top side with the Nonwoven Fabric A coated with the above-mentioned SBS in the same manner as above at a basis weight of 25 g/m$^2$, and thereafter heated-fused with a laminating machine (manufactured by HASHIMA CO., LTD., straight linear fusing press HP-600LF) of which heating temperature was set at 100°C to integrate, to give a water-absorbent sheet structure.
The resulting water-absorbent sheet structure was cut into a given size to perform the above-mentioned various measurements and evaluations. The results are shown in Table 3.

(Comparative Example 2)

The same procedures as in Example 2 were carried out except that a fibrous substrate to be used was changed to the Nonwoven Fabric A in Example 2, to give a water-absorbent sheet structure.

The resulting water-absorbent sheet structure was cut into a given size with an absorbent layer using a water-absorbent resin A on an upper side (primary absorbent layer) to perform the above-mentioned various measurements and evaluations. The results are shown in Table 3.

(Comparative Example 3)

The same procedures as in Example 2 were carried out except that the nonwoven fabric and the fibrous substrate to be used were all changed to the SMS nonwoven fabric (fiber: polypropylene, void ratio: 90%, basis weight: 17 g/m$^2$, thickness: 190 $\mu$m, degree of hydrophilicity: 3 or less; referred to as "Nonwoven Fabric E") in Example 2, to give a water-absorbent sheet structure.

The resulting water-absorbent sheet structure was cut into a given size with an absorbent layer using a water-absorbent resin A on an upper side (primary absorbent layer) to perform the above-mentioned various measurements and evaluations. The results are shown in Table 3.

(Comparative Examples 4 and 5)

The same procedures as in Example 1 were carried out except that a fibrous substrate to be used was changed to Nonwoven Fabric C, and that the content of the adhesive was changed to as described in Table 2 in Example 1, to give each of water-absorbent sheet structures.

The water-absorbent sheet structures obtained in Comparative Examples 4 and 5 were cut into a given size with an absorbent layer using a water-absorbent resin A on an upper side (primary absorbent layer) to perform the above-mentioned various measurements and evaluations. The results are shown in Table 3.

(Comparative Examples 6 and 7)

The same procedures as in Example 3 were carried out except that the content of the water-absorbent resins A and B and the adhesive to be used were changed to those listed in Table 2 in Example 3, to give each of water-absorbent sheet structures.

The water-absorbent sheet structures obtained in Comparative Examples 6 and 7 were cut into a given size with an absorbent layer using a water-absorbent resin A on an upper side (primary absorbent layer) to perform the above-mentioned various measurements and evaluations. The results are shown in Table 3.

Various properties of the nonwoven fabrics and the fibrous substrates used in Examples and Comparative Examples are shown in Table 1.

[Table 1]

Table 1

| Abbreviation | Fibers | Void Ratio (%) | Basis Weight (g/m$^2$) | Thickness ($\mu$m) | Degree of Hydrophilicity |
|---|---|---|---|---|---|
| Water-Absorbent Paper | Pulp | 95 | 25 | 350 | 55 |
| Nonwoven Fabric A | Polypropylene | 90 | 13 | 150 | 16 |
| Nonwoven Fabric B | Rayon/Polyethylene Terephtalate | 92 | 35 | 300 | 38 |
| Nonwoven Fabric C | Polypropylene/Polyethylene | 92 | 23 | 150 | 33 |
| Nonwoven Fabric D | Polypropylene/Polyethylene | 97 | 20 | 820 | 12 |
| Nonwoven Fabric E | Polypropylene | 90 | 17 | 190 | 3 or less |

[Table 2]

Table 2

| Ex. No. | Nonwoven Fabrics | | Fibrous Substrate | Water-Absorbent Resin (g/m²) | | | Adhesive (g/m²) | | |
|---|---|---|---|---|---|---|---|---|---|
| | Upper Side | Lower Side | Kinds | Primary | Secondary | Ratio* | Primary | Secondary | Content Ratio** |
| Ex. 1 | Nonwoven Fabric A | Nonwoven Fabric A | Water-Absorbent Paper | 200 | 50 | 80/20 | 40 | 18 | 0.23 |
| Ex. 2 | Nonwoven Fabric A | Nonwoven Fabric A | Nonwoven Fabric B | 200 | 50 | 80/20 | 30 | 20 | 0.20 |
| Ex. 3 | Nonwoven Fabric A | Nonwoven Fabric A | Nonwoven Fabric C | 200 | 50 | 80/20 | 30 | 20 | 0.20 |
| Ex. 4 | Nonwoven Fabric A | Nonwoven Fabric A | Nonwoven Fabric D | 200 | 50 | 80/20 | 30 | 20 | 0.20 |
| Ex. 5 | Nonwoven Fabric C | Nonwoven Fabric A | Nonwoven Fabric C | 200 | 50 | 80/20 | 30 | 20 | 0.20 |
| Ex. 6 | Nonwoven Fabric D | Nonwoven Fabric D | Nonwoven Fabric D | 200 | 50 | 80/20 | 30 | 20 | 0.20 |
| Ex. 7 | Nonwoven Fabric A | Nonwoven Fabric A | Nonwoven Fabric C | 600 | 120 | 83/17 | 80 | 30 | 0.15 |
| Ex. 8 | Nonwoven Fabric A | Nonwoven Fabric A | Nonwoven Fabric C | 150 | 150 | 50/50 | 20 | 35 | 0.18 |
| Comp. Ex. 1 | Nonwoven Fabric A | Nonwoven Fabric A | - | 250 | - | - | 50 | - | 0.20 |
| Comp. Ex. 2 | Nonwoven Fabric A | Nonwoven Fabric A | Nonwoven Fabric A | 200 | 50 | 80/20 | 30 | 20 | 0.20 |
| Comp. Ex. 3 | Nonwoven Fabric E | Nonwoven Fabric E | Nonwoven Fabric E | 200 | 50 | 80/20 | 30 | 20 | 0.20 |
| Comp. Ex. 4 | Nonwoven Fabric A | Nonwoven Fabric A | Nonwoven Fabric C | 200 | 50 | 80/20 | 5 | 3 | 0.03 |
| Comp. Ex. 5 | Nonwoven Fabric A | Nonwoven Fabric A | Nonwoven Fabric C | 200 | 50 | 80/20 | 400 | 150 | 2.20 |
| Comp. Ex. 6 | Nonwoven Fabric A | Nonwoven Fabric A | Nonwoven Fabric C | 50 | 20 | 71/29 | 20 | 10 | 0.43 |
| Comp. Ex. 7 | Nonwoven Fabric A | Nonwoven Fabric A | Nonwoven Fabric C | 30 | 60 | 33/67 | 20 | 20 | 0.44 |

*) Ratio of the primary absorbent layer to the secondary absorbent layer, i.e. primary/secondary, of the water-absorbent resin (weight ratio).
**) Content proportion of the adhesive, i.e. the content based on the water-absorbent resin on mass basis.

[Table 3]

Table 3

| Ex. No. | Thickness (mm) | Liquid Permeation Rate Under Load (sec) | | | Amount of Re-wet (g) | Slope Leakage Index | | | Shape Retaining Ability |
|---|---|---|---|---|---|---|---|---|---|
| | | 1 | 2 | Total | | LW1 | LW2 | Total | |
| Ex. 1 | 1.3 | 353 | 324 | 677 | 34 | 0 | 0 | 0 | B |
| Ex. 2 | 1.2 | 451 | 346 | 797 | 25 | 1 | 0 | 10 | A |
| Ex. 3 | 1.2 | 401 | 310 | 711 | 21 | 3 | 0 | 20 | A |
| Ex. 4 | 1.7 | 322 | 330 | 662 | 18 | 3 | 2 | 32 | A |
| Ex. 5 | 1.3 | 351 | 304 | 655 | 19 | 4 | 2 | 42 | A |
| Ex. 6 | 2.4 | 264 | 328 | 592 | 15 | 6 | 2 | 62 | A |
| Ex. 7 | 2.2 | 293 | 303 | 596 | 6 | 0 | 0 | 0 | A |
| Ex. 8 | 1.2 | 433 | 319 | 752 | 20 | 0 | 0 | 0 | A |
| Comp. Ex. 1 | 1.1 | 747 | 593 | 1,340 | 25 | 22 | 11 | 231 | A |
| Comp. Ex. 2 | 1.1 | 608 | 532 | 1,140 | 27 | 2 | 0 | 20 | A |
| Comp. Ex. 3 | 1.1 | 855 | 587 | 1,442 | 20 | 83 | 67 | 897 | A |
| Comp. Ex. 4 | 1.2 | 381 | 330 | 711 | 19 | 0 | 0 | 0 | C |
| Comp. Ex. 5 | 1.2 | 832 | 573 | 1,405 | 28 | 40 | 23 | 423 | A |
| Comp. Ex. 6 | 1.1 | 742 | 557 | 1,299 | 43 | 46 | 38 | 498 | A |
| Comp. Ex. 7 | 1.1 | 813 | 588 | 1,401 | 39 | 12 | 56 | 176 | A |

It could be seen from the above results that the water-absorbent sheet structures of Examples had faster liquid permeation rates under load, smaller amounts of re-wet, smaller slope liquid leakages, more favorable liquid absorbent properties, and excellent shape retaining ability, as compared to those of Comparative Examples. In addition, it could be seen that when inside of the water-absorbent sheet structure after performing slope leakage test was confirmed, the entire of the absorbent layer was evenly swollen, and the gel-blocking phenomena did not take place.

On the other hand, when Comparative Examples were studied, in a case where the absorbent layer was a single layer (Comparative Example 1), both the evaluations of the liquid permeation rate under load and the slope leakage index were low. In a case where one having a low void ratio was used as a fibrous substrate (Comparative Example 2), the liquid permeation rate under load was slow and the water-absorbent capacity was not sufficient. In a case where one having low hydrophilicity and void ratio was used as the nonwoven fabric and the fibrous substrate (Comparative Example 3), the water-absorbent capacity was not sufficient, and especially slope leakage index was likely to be high. In a case where a ratio of an adhesive used based on a water-absorbent resin was small (Comparative Example 4), the water-absorbent capacity was in an operable level, but had disadvantages in strength, so that the water-absorbent sheet structure cannot be said to be sufficiently satisfactory. Conversely, in a case where a ratio of the adhesive used based on a water-absorbent resin was high (Comparative Example 5), the swelling of the water-absorbent resin upon liquid absorption was inhibited, thereby causing gel-blocking phenomena, so that both the evaluations of the liquid permeation rate under load and the slope leakage index were low. In a case where an amount of a water-absorbent resin used was small (Comparative Example 6), the water-absorbent properties of an entire water-absorbent sheet structure were lowered, so that all the evaluations of the liquid permeation rate under load, the amount of re-wet, and the slope leakage index were low. In a case where an amount of water-absorbent resin used was small and a ratio of a resin used in the secondary absorbent layer was high (Comparative Example 7), the liquid permeation rate under load was further worsened as compared to Comparative Example 6. This causation is considered to be due to the fact that the ratio of resin used in the secondary absorbent layer was high, thereby causing gel-blocking phenomena in the secondary absorbent layer.

INDUSTRIAL APPLICABILITY

The water-absorbent sheet structure of the present invention can be used for absorbent articles in hygienic material fields, agricultural fields, construction material fields, and the like, among which the water-absorbent sheet structure can be suitably used for absorbent articles in hygienic material fields.

EXPLANATION OF NUMERICAL SYMBOLS

10    adhesive
11    water-absorbent sheet structure
12    water-absorbent resin
13    primary absorbent layer
14    water-absorbent resin
15    secondary absorbent layer
16    fibrous substrate
17    nonwoven fabric
18    nonwoven fabric
19    adhesive
21    acrylic plate
22    acrylic plate
23    water-absorbent sheet structure
31    stand
32    acrylic plate
33    absorbent article
34    dropping funnel
35    balance
36    tray

**Claims**

1.   A water-absorbent sheet structure comprising a structure in which an absorbent layer comprising a water-absorbent resin and an adhesive is sandwiched with nonwoven fabrics from an upper side and a lower side of the absorbent layer, wherein the water-absorbent sheet structure has a structure in which the absorbent layer is separated in divided parts of a primary absorbent layer and a secondary absorbent layer with a fibrous substrate having a void ratio of from 91 to 99%, and wherein the water-absorbent resin is contained in an amount of from 100 to 1,000 g/m$^2$, and wherein the adhesive is contained in a proportion of 0.05 to 2.0 times the amount of the water-absorbent resin contained (mass basis).

2.   The water-absorbent sheet structure according to claim 1, wherein the fibrous substrate has a basis weight of 15 g/m$^2$ or more.

3.   The water-absorbent sheet structure according to claim 1 or 2, wherein the fibrous substrate is a nonwoven fabric made of rayon-containing synthetic fiber and/or a nonwoven fabric made of hydrophilically treated synthetic fibers

4.   The water-absorbent sheet structure according to any one of claims 1 to 3, wherein the nonwoven fabrics are nonwoven fabrics made of at least one fiber selected from the group consisting of rayon fibers, polyolefin fibers, and polyester fibers.

5.   The water-absorbent sheet structure according to any one of claims 1 to 4, wherein the adhesive is at least one member selected from the group consisting of ethylene-vinyl acetate copolymer adhesives, styrene-based elastomer adhesives, polyolefin-based adhesives, and polyester-based adhesives.

**6.** The water-absorbent sheet structure according to any one of claims 1 to 5, wherein the water-absorbent sheet structure satisfies all the properties of the following (A) to (C):

    (A) the water-absorbent sheet structure having a thickness of 4 mm or less, in a dry state,
    (B) a liquid permeation rate under load of 1,000 seconds or less, and
    (C) a leakage index of 200 or less.

**7.** An absorbent article comprising the water-absorbent sheet structure as defined in any one of claims 1 to 6, sandwiched between a liquid-permeable sheet and a liquid-impermeable sheet.

[Figure 1]

FIG. 1

[Figure 2]

FIG. 2

[Figure 3]

FIG. 3

[Figure 4]

**FIG. 4**

[Figure 5]

**FIG. 5**

## INTERNATIONAL SEARCH REPORT

| | International application No. |
|---|---|
| | PCT/JP2011/059626 |

**A. CLASSIFICATION OF SUBJECT MATTER**
*B32B27/12*(2006.01)i, *A61F13/15*(2006.01)i, *A61F13/49*(2006.01)i, *A61F13/53* (2006.01)i, *B32B5/26*(2006.01)i

According to International Patent Classification (IPC) or to both national classification and IPC

**B. FIELDS SEARCHED**

Minimum documentation searched (classification system followed by classification symbols)
B32B1/00-43/00, A61F13/00-13/84

Documentation searched other than minimum documentation to the extent that such documents are included in the fields searched
Jitsuyo Shinan Koho        1922-1996    Jitsuyo Shinan Toroku Koho    1996-2011
Kokai Jitsuyo Shinan Koho    1971-2011    Toroku Jitsuyo Shinan Koho    1994-2011

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| P,X | WO 2010/076857 A1 (Sumitomo Seika Chemicals Co., Ltd.), 08 July 2010 (08.07.2010), claims; paragraphs [0030] to [0039], [0047] to [0049]; examples; drawings (Family: none) | 1-7 |
| P,X | WO 2010/082373 A1 (Sumitomo Seika Chemicals Co., Ltd.), 22 July 2010 (22.07.2010), claims; paragraphs [0039] to [0047], [0056] to [0058]; examples; drawings (Family: none) | 1-7 |

[X] Further documents are listed in the continuation of Box C.    [ ] See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 25 July, 2011 (25.07.11) | 02 August, 2011 (02.08.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2011/059626 |

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 2010/143635 A1 (Sumitomo Seika Chemicals Co., Ltd.), 16 December 2010 (16.12.2010), claims; paragraphs [0032] to [0044], [0046] to [0048]; examples; drawings (Family: none) | 1-7 |
| P,X | WO 2011/043256 A1 (Sumitomo Seika Chemicals Co., Ltd.), 14 April 2011 (14.04.2011), claims; paragraphs [0023] to [0033], [0054] to [0056], [0065] to [0067]; examples; drawings (Family: none) | 1-7 |
| A | JP 6-315501 A (Sekisui Plastics Co., Ltd.), 15 November 1994 (15.11.1994), claims; examples; drawings (Family: none) | 1-7 |
| A | JP 7-327899 A (Shoichi MARUYAMA et al.), 19 December 1995 (19.12.1995), claims; examples; drawings (Family: none) | 1-7 |
| A | JP 6-59039 U (Sekisui Plastics Co., Ltd.), 16 August 1994 (16.08.1994), claims; drawings (Family: none) | 1-7 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI9510889 B
- JP HEI8057311 B
- JP HEI9253129 B
- JP HEI7051315 B
- JP 2002325799 A

- JP 2000238161 A
- JP 2001158074 A
- JP 2001096654 A
- JP 2003011118 A
- JP HEI2048944 B

**Non-patent literature cited in the description**

- *JAPAN TAPPI Test Method No. 68,* 2000